# EUROPEAN PATENT APPLICATION

(11) **EP 4 668 225 A1**
(43) Date of publication of application: **24.12.2025**
(21) Application number: 24183831.7
(22) Date of filing: 21.06.2024
(51) Int. Cl.: G06V 10/26, G06T 7/00, G06T 7/10, G16H 30/20, A61B 6/03

(54) **AUTOMATIC DETECTION OF SMA AND IMA ARTERIES AND ITS TERMINAL BRANCHES**

(71) Applicant: Siemens Healthineers AG, 91301 Forchheim (DE)
(72) Inventor: CHANDOLIA, Hermant, 560100 Bangalore, Karnataka (IN); M, Muniraju, 562106 Bangalure, Karnataka (IN); R, Jayanna, 560032 Bangalore (IN)
(74) Representative: HKW Intellectual Property PartG mbB

(57) **Abstract**

The invention relates to a method, an apparatus, a system and a computer program product for identifying at least one blood vessel represented in image data, the method comprising accessing the image data, passing the image data to a trained artificial intelligence unit being trained to perform a multi-class segmentation on the image data to identify the at least one blood vessel and outputting an indication associated with the identified at least one blood vessel.

## Description

The present invention relates to a method for identifying at least one blood vessel represented in image data, an apparatus for identifying at least one blood vessel represented in image data, a system for identifying at least one blood vessel represented in image data and to a computer program product that is configured to carry out the aforementioned method when executed.

Medical imaging has provided a powerful tool for diagnosing abnormalities in a body of a patient for many years and has developed to represent a standard procedure in state-of-the-art medical diagnostics. Medical imaging made it possible to support a non-invasive diagnosis, by a physician, based on images that were captured of certain parts of a body of a patient. Said images were, e.g., captured by means of X-ray imaging, computed tomography (CT) imaging, positron emission tomography (PET) imaging, ultrasonic imaging, etc.

The imaging quality of these known imaging methods has drastically improved over the last decades supporting the accurate identification of ever decreasing abnormalities. The imaging quality has further improved in that that the imaging quality of imaging devices as such has improved, allowing a precise capturing of, e.g., X-rays which have penetrated the body of the patient with an improved contrast ration (as compared to respective devices used decades ago) and suppressed blurring effects. These improvements of the imaging quality as such have advantageously contributed to a general improvement of medical imaging as such and increased the constant demand for medical imaging even further.

However, even nowadays, the captured images are, to a wide extent, still analyzed manually by an experienced physician. This is time and resource consuming and leads to rather high costs of medical imaging which need to be compensated by already weakened healthcare systems and are accompanied by prolonged waiting times for the patients requiring a medical imaging-based diagnosis.

What is more, especially in CT imaging, many images are captured sequentially wherein each of the images may be associated with a certain layer depth (along a cross-section) of the human body that is imaged. In other words, in CT imaging, a 3-dimenisonal image of the body of the patient may be acquired by capturing a plurality of images. This plurality of images may then be required to be studied manually by a respective physician to derive a potential diagnosis.

This procedure may be seen as reliable for large scale anomalies, such as, e.g., ruptures of bones. However, the accuracy and reliability of the analysis of medical images may deteriorate when anomalies need to be identified that appear on a smaller scale as compared to, e.g., bones. Given the large number of, e.g., CT images that needs to be studied and given the stressful working days physicians find themselves in everyday, the risk that an anomaly is not manually identified raises, e.g., due to a wearied physician. The consequence may be that a potentially present (anatomic) anomaly is not diagnosed at all or only diagnosed at a late stage of an underlying disease with respectively disadvantageously affected chances for being cured.

That is more, acquired images may sometimes be subject to automized processing (e.g., to increase the contrast of the respective image). However, this sometimes comes with the price of affecting small scale anatomical structures in a way that may not be easily identifiably by a physician anymore.

The detection of such small anomalies plays a dominant role when small blood vessels need to be investigated. These small-scale structures can oftentimes not be reliably identified based on currently existing (mostly manual) methods of studying medical images. However, even though some blood vessels may be small with respect to their dimensions, they may nevertheless be subject to severe and threatening diseases.

Therefore, there is still a need to improve the analysis of medical images, in particular, when it comes to the analysis of small-scale structures, such as, e.g., blood vessels, which is currently not yet fulfilled in a satisfying manner based on the presently known and applied procedures.

It is an object of the present invention to further improve the identifying of small-scale structures, such as, e.g., blood vessels, in image data.

According to a first aspect, a method for identifying at least one blood vessel represented in image data is provided. The method comprises accessing the image data and passing the image data to a trained artificial intelligence unit being trained to perform a multi-class segmentation on the image data to identify the at least one blood vessel. Moreover, the method comprises outputting an indication associated with the identified at least one blood vessel.

Passing the image data to a trained artificial intelligence unit that is trained to perform a multi-class segmentation may support a fully automized analysis of the acquired image data. That is, the time a physician is required to spend with analyzing the respective image data may drastically be reduced such that the associated overall costs associated with the medical imaging may also be reduced. What is more, the accuracy and reliability of the identification of certain parts of the human body (e.g., by means of the multi-class segmentation) may be improved and kept at a constantly high level as fatigue-related effects of a physician which may decrease the reliability of such a manual analysis may be reduced (e.g., as respective blood vessels may be missed when scrolling through the image data). The outputting of an indication associated with the identified at least one blood vessel may support the association of an anatomic structure in the image data with a certain blood vessel, e.g., by a physician who is provided with the indication. Therefore, an improved analysis of image data may be provided and the reliability and accuracy of finding blood vessels may be improved.

According to an embodiment, the image data may represent medical image data.

In some cases, accessing the image data comprises accessing a storage of a device that is configured to acquire the image data. Additionally or alternatively, accessing the image data may comprise receiving the image data from another device and may further comprise processing (e.g., decompressing) the received image data such that it may be passed to the trained artificial intelligence unit.

According to a further embodiment, the multi-class segmentation may facilitate the simultaneous identification of a plurality of blood vessels in the image data provided to the trained artificial intelligence unit such that the indication that may be output indicates a plurality of (different) blood vessels in the image data. As a result, the spectrum of possible applications of the automized identification of blood vessels, according to aspects of the present invention, may be extended.

In some embodiments, the application of the aforementioned identification of blood vessels is not exclusively limited to blood vessels only but may also be applied to the identification of certain bones, organs in image data supplied to the trained artificial intelligence unit if the trained artificial intelligence unit is trained accordingly.

According to a further embodiment, the accessing of the image data may comprise retrieving the image data from a remote entity.

The remote entity may be physically separated from a unit that is adapted to acquire the image data and which may thus be physically separated from a patient.

The remote entity may be a remote server (connected to the artificial intelligence unit over a local area network, the internet, etc.), a cloud infrastructure, a hospital information system, a practice of a physician, a remotely deployed unit for acquiring image data, etc.

What is more, the remote entity may be adapted to provide data safety means (e.g., a RAID system).

This may allow a retrospective analysis of acquired image data such that potential blood vessels that may not have been identified in a previous analysis (e.g., by a physician) may now be identified by means of aspects of the pending application. What is more, a spatially separated storage of image data and analysis of the image data by means of the trained artificial intelligence unit may avoid that the trained artificial intelligence unit is required to provide an extensive and cost-effective data storage unit. In other words, the artificial intelligence unit may be optimized for analyzing purposes only.

According to a further embodiment, the image data may represent abdominal image data.

Abdominal image data may be understood as image data that represents image data of a part of the body of the patient that is arranged between a thorax (chest) and a pelvis of the body of the patient.

Since several small blood vessels are located in the abdominal part of the body of the patient, the identification of exactly these small blood vessels may advantageously be supported by aspects of the present invention.

According to a further embodiment, the at least one blood vessel may be a superior mesenteric artery, SMA, and/or an inferior mesenteric artery, IMA, and/or a respective terminal branch.

The respective terminal branch may be a terminal branch of the SMA or of the IMA.

As these vessels are of particular importance for the body of the patient and since these vessels may oftentimes be involved in several pathology conditions, but oftentimes ignored because of their small dimensions or because they are overlapped with other organs, aspects of the pending application may allow their improved identification.

According to a further embodiment, the image data may be computed tomography, CT, image data.

The CT image data may be acquired by a photon counting CT scanner, which may improve the image quality of the obtained CT image data.

According to a further embodiment, the trained artificial intelligence unit may be based at least in part on a U-Net architecture.

A U-Net architecture represents a fully convolutional neural network that may specifically be used for image segmentation and may also be called semantic segmentation.

The usage of a U-Net architecture for aspects of the pending invention may improve the efficiency (e.g., with respect to processing time) and accuracy of the identifying of the at least one blood vessel.

According to a further embodiment, the U-Net architecture may be trained by using skip connections.

Skip connections during training may be understood as a skipping of some layers in a (convolutional) neural network and such that the output of one layer is not directly passed to the input of a subsequent layer (as seen in a depth direction of the neural network) but to a layer that is arranged (at least) subsequent to the subsequent layer such that at least one layer is skipped.

Using skip connections may advantageously support an efficient and fast learning procedure. What is more, the usage of skip connections may reduce the amount of required training data for training the neural network while still allowing a reliable accuracy of the trained network.

According to a further embodiment, the outputting may comprise visually masking the identified blood vessel in the image data.

The visual masking may be provided as a highlighting of a contour of the identified at least one blood vessel. The highlighting of a contour may be understood as drawing a line (e.g., a dashed-lined, a solid line, etc.) about the identified at least one blood vessel such that the line is preferably circumferent to the identified at least one blood vessel in the analyzed image data.

Additionally or alternatively, the visually masking may comprise depicting at least one (colored) arrow in the image data wherein the at least one arrow points to (and thus indicates) the identified at least one blood vessel.

Additionally or alternatively, the visually masking may be a (colored) shading of the identified at least one blood vessel.

The visually masking may support a subsequent identification of the least one blood vessel, identified by the trained artificial neural network unit, by a physician. This may decrease the risk that a physician may miss the at least one blood vessel.

According to a further embodiment, the outputting may comprise outputting a dimension of the identified at least one blood vessel.

The dimension may be referred to as a metric measure of, e.g., a longitudinal extension of the identified at least one blood vessel in the image data (e.g., in mm and/or in cm and/or in m). Alternatively or additionally, the dimension may be referred to as a thickness of the identified at least one blood vessel (e.g., as seen in a cross-sectional view).

The outputting of the dimension of the identified at least one blood vessel may provide a physician with additional metadata and/or background information regarding the identified at least one blood vessel.

According to a further embodiment, the method may further comprise detecting an anomaly in the at least one blood vessel based at least in part on the outputting.

An anomaly may be understood as an anatomic artefact in the identified at least one blood vessel such as, e.g., a change of a property of the blood vessel which is typically not expected. That is, an anomaly may, e.g., refer to an (local) occlusion of the blood vessel and/or a (local) widening of the blood vessel. Additionally or alternatively, the anomaly may refer to a local rupturing of the blood vessel. In particular, an occlusion of the SMA and/or IMA may lead to a reduced oxygenation of the stomach of the patient with respective detrimental effects.

The (automatic) detecting of an anomaly may support a reliable and accurate diagnosis of a potential disease, by a physician, and may thus contribute to increased chances for successfully curing the patient.

According to a second aspect, a computer program product is suggested which comprises instructions which, when the program is executed by a computer, cause the computer to carry out the method as outlined above.

A computer program means, may be embodied as a memory card, USB stick, CD-ROM, DVD or as a file which may be downloaded from a server in a network. For example, such a file may be provided by transferring the file comprising the computer program product from a wireless communication network.

One or more algorithms/computer programs described herein may be embodied directly in hardware, in a software module executed by a processor, or in a combination of the two. A software module may reside in Random Access Memory (RAM), flash memory, Read Only Memory (ROM), Electrically Programmable ROM (EPROM), Electrically Erasable Programmable ROM (EEPROM), registers, hard disk, a removable disk, a CD-ROM, or any other form of storage medium known in the art. An exemplary storage medium is coupled to the processor so the processor may read information from, and writes information to, the storage medium. In the alternative, the storage medium may be integral to the processor. The processor and the storage medium may reside in an application-specific integrated circuit (ASIC). In the alternative, the processor and the storage medium may reside as discrete components in any component of a mobile computing platform.

According to a third aspect an apparatus for identifying at least one blood vessel represented in image data is suggested. The apparatus comprises an accessing unit for accessing the image data and a passing unit for passing the image data to a trained artificial intelligence unit that has been trained to perform a multi-class segmentation on the image data to identify the at least one blood vessel. Moreover, the apparatus comprises an outputting unit for outputting an indication associated with the identified at least one blood vessel.

In an embodiment, the apparatus may further comprise an execution unit for executing the method as outlined above.

According to a further embodiment, the execution unit may comprise any means that are required for executing the respective method and their respective method steps.

According to a further embodiment, the apparatus may further comprise an execution unit for executing the computer program as outlined above.

According to a fourth aspect, a system for identifying at least one blood vessel represented in image data is suggested. The system comprises a remote storage entity which is configured to store the image data and comprises the apparatus as outlined above.

The embodiments and features described with reference to the apparatus and/or the system of the present invention apply, mutatis mutandis, to the method of the present invention and vice versa.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention. In other words, it will be apparent for the person skilled in the art that different embodiments described herein may be combined with each other.

Independent of the grammatical term usage, individuals with male, female or other gender identities are included within the term.

Further embodiments, features and advantages of the present invention will become apparent from the subsequent description and dependent claims, taken in conjunction with the accompanying drawings, in which:
Fig. 1 shows a schematic illustration of an artery system in an abdominal region of a patient;
Figs. 2A and 2B show examples of image data of SMA/IMA arteries in the abdominal region of the patient according to Fig. 1;
Fig. 3 shows a schematic illustration of a clinical workflow for identifying the SMA and IMA arteries according to Figs. 2A or 2B;
Fig. 4 shows a schematic illustration of a proposed clinical workflow for identifying at least one blood vessel;
Fig. 5 shows a schematic illustration of the interplay of an encoder and a decoder as it may be used for identifying the at least one blood vessel;
Figs. 6A and 6B show examples of image data of the SMA/IMA arteries wherein the identified SMA/IMA arteries are visually masked;
Fig. 7 shows a schematic illustration of a workflow for a method for identifying at least one blood vessel;
Fig. 8 shows a schematic illustration of an apparatus for identifying at least one blood vessel; and
Fig. 9 shows a schematic illustration of a system for identifying at least one blood vessel.

In the Figures, reference numerals designate like or functionally equivalent elements, unless otherwise indicated.

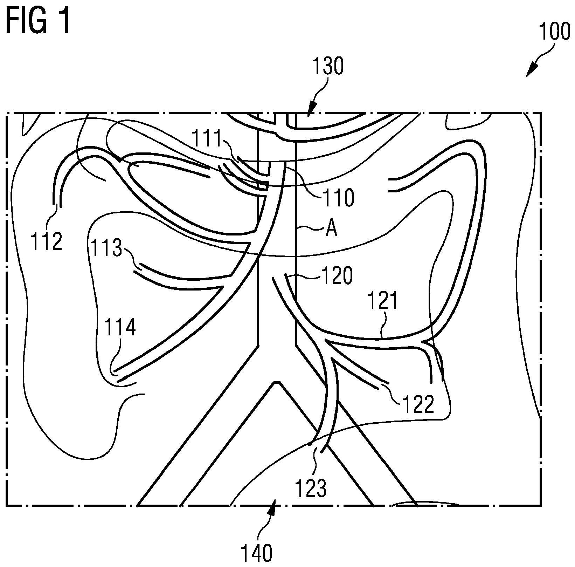

More specifically, Fig. 1 shows a schematic illustration of an artery system 100 in an abdominal region of a patient.

The artery system 100 comprises an abdominal aorta A from which a superior mesenteric artery (SMA) 110 located in an upper region of the abdominal region of the patient (e.g., a region that is closer to a heart 130 of the patient than it is to a pelvis 140 of the patient) and an inferior mesenteric artery (IMA) 120 located in a lower region of the abdominal region of the patient (e.g., a region that is closer to the pelvis 140 of the patient than it is to the heart 130 of the patient) originate. The SMA 110 originates from a portion of the abdominal aorta A that lies closer to the heart 130 than a portion of the abdominal aorta A from which the IMA 120 originates.

The SMA 110 and the IMA 120 extend lateral to the abdominal aorta A at a level of a third lumbar vertebrae behind a third part of a duodenum.

The SMA 110 and the IMA 120 are arteries (as representatives of blood vessels) in the abdomen of the patient which provide oxygenated blood and nutrients to the intestines.

The SMA 110 typically possesses a length of 3 to 7 mm and a diameter of 5 to 7.3 mm.

The IMA 120 typically possesses a length of 10 to 80 mm and a diameter of less than 3 mm.

Each of the SMA 110 and the IMA 120 may comprise several terminal branches 111-114 and 121-123, respectively.

The terminal branches 111-114, 121-123 may be understood as the terminal branches of the SMA 110 and the IMA 120 which may supply a colon of the patient and which are linked together by a continuous arterial circle or arcade along a mesenteric border, known as the marginal artery of Drummond (not shown in Fig. 1).

For example, the terminal branches of the SMA 110 may be an inferior pancreaticoduodenal artery (IPD) 111, middle colic (MC) 112, right colic (RC) 113 and ileocolic (IC) 114. The terminal branches 111-114, may branch from the SMA 110 along a longitudinal extension of the SMA 110 towards a pelvis 130 of the patient in the order stated above.

The terminal branches of the IMA 120 may be a left colic (LC) 121, sigmoid branches 122 and a superior rectal branch 123. The terminal branches 121-123 may branch from the IMA 120 along a longitudinal extension of the IMA 120 towards a pelvis 130 of the patient in the order stated above.

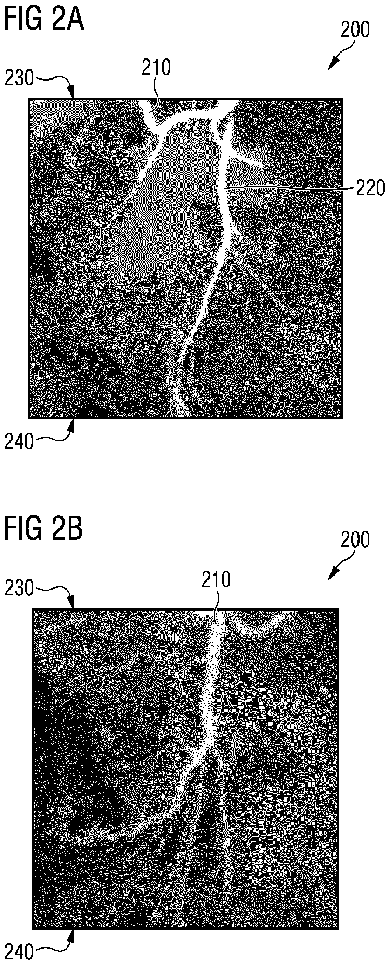

Figs. 2A and 2B show exemplary image data 200 of the SMA 210 and the IMA 220 arteries in the abdominal region of the patient. The SMA 210 and the IMA 220 may correspond to the SMA 110 and the IMA 220, respectively, as described with reference to Fig. 1, above. The heart 230 and the pelvis 240 may correspond to the heart 130 and the pelvis 140 as described with reference to Fig. 1, above.

Fig. 2A shows a first exemplary image data 200 of the SMA 210 that extends from an upper part of the abdominal region that lies closer to the heart 230 of the patient than it lies to the pelvis 240 of the patient to a lower abdominal region that lies closer to the pelvis 240 of the patient than it lies to the heart 230 of the patient.

Moreover, Fig. 2A shows the IMA 220 that extends from an upper part of the abdominal region that lies closer to the heart 230 of the patient than it lies to the pelvis 240 of the patient to a lower abdominal region that lies closer to the pelvis 240 of the patient than it lies to the heart 230 of the patient.

Fig. 2B shows a second exemplary image data of the SMA 210 that extends from an upper part of the abdominal region that lies closer to the heart 230 of the patient than it lies to the pelvis 240 of the patient to a lower abdominal region that lies closer to the pelvis 240 of the patient than it lies to the heart 230 of the patient.

The exemplary image data 200 depicted in Figs. 2A and 2B was taken by a CT scanner wherein a contrast agent has been added to the SMA 210 and the IMA 220 to improve the contrast of the SMA 210 and the IMA 220 with respect to the surrounding tissue in the acquired image data.

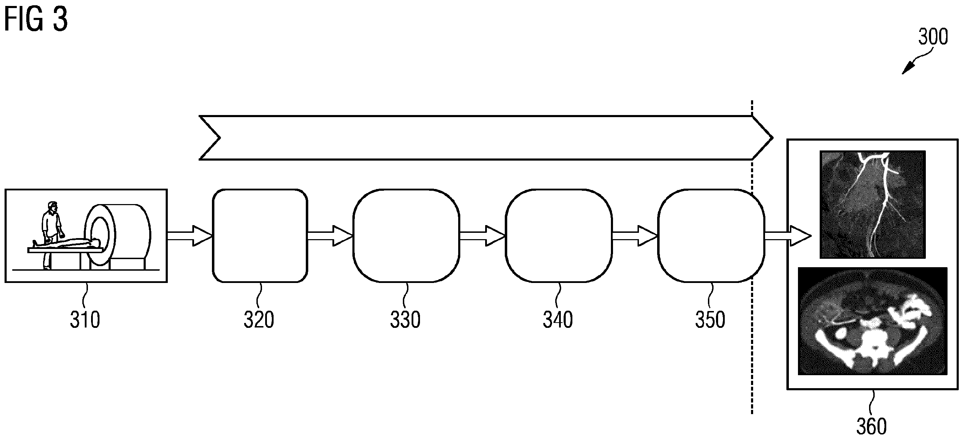

Fig. 3 shows an exemplary clinical workflow 300 for identifying the SMA 110, 210 and/or the IMA 120, 220 as discussed with reference to Figs. 1, 2A and 2B, above.

First, a patient is prepared 310 for an abdomen scan after a contrast agent was preferably administered to the patient (e.g., orally). The abdomen scan is preferably a CT scan but may in some cases also be an X-ray scan. If the abdomen scan is carried out as a CT scan, multiple images of the patient may be acquired, represented as respective image data, wherein each of the images corresponds to a different layer of the body (acquired as respective slices) of the patient (e.g., taken along an axial direction) such that the totality of acquired images corresponds to a 3-dimenisonal scan of the body of the patient.

Afterwards, a multiphase reconstruction 320 of the scan of the body is performed, preferably in post-processing. In an example, the acquired image data may be processed such that subsequently acquired slices of the body of the patient may be obtained (e.g., along an axial direction (e.g., as a cross-sectional view taken along an axis from a head of the patient to a foot of the patient) of the patient) with a respective thickness of each of the slices of, e.g., 1 mm.

Subsequently, the individual layers of the scan may be loaded 330 into a multiphase reconstruction to obtain a 3-dimensional reconstruction of the patient. Consequently, the SMA 110, 210 and IMA 120, 220 may be seen from various directions and with different orientations.

Afterwards, the multiphase reconstruction may be changed 340 to a maximum intensity projection to obtain a better visualization of vessels.

Then, the maximum intensity projection may be changed 350 to a volume reconstruction (VRT) view in which bone structures and/or any other anatomic structures (e.g., organs) in the processed image data may be removed such that only vessel structures remain in the processed image data and such that a 360° view of the vessel structures is obtained.

In this conjunction, it is stressed that applying VRT presets to the image processing may oftentimes lead to an unintended removal of small vessels (e.g., terminal branches) from the SMA 110, 210 and/or IMA 120, 220. This may lead to the disadvantageous effect that the respectively removed vessels may not be identified by a physician anymore.

It may be appreciated that steps 330-350 may be performed by a physician (e.g., a radiologist) or at least in the presence of a physician.

This procedure may finally enable the identification 360 of the SMA 110, 210 and/or the IMA 120, 220.

It is emphasized that the aforementioned method steps 330 to 350 are mostly required to be carried out by a radiologist manually.

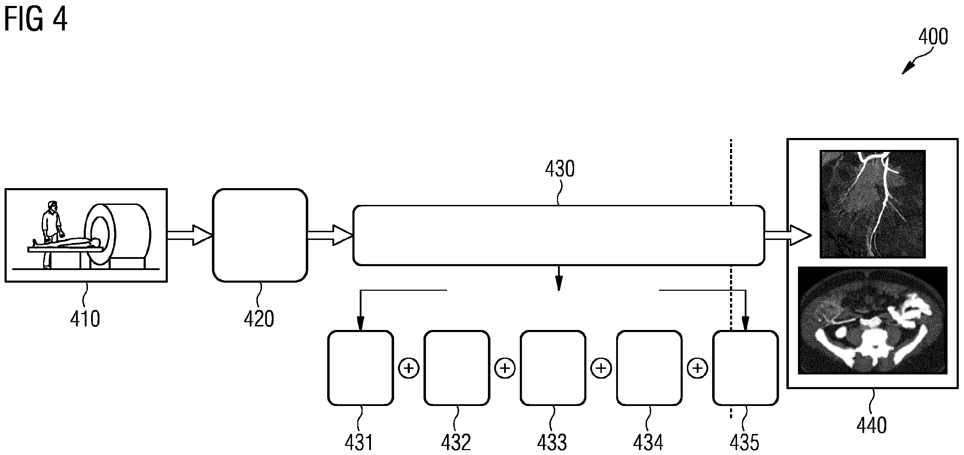

Fig. 4 shows a schematic illustration of a proposed clinical workflow 400 for identifying at least one blood vessel according to aspects of the present invention.

The workflow 400 starts with the preparation 410 of a patient for an abdomen scan. The preparation 410 may comprise administering a contrast agent to the patient (e.g., orally).

Afterwards, a multiphase reconstruction scan is performed 420. The multiphase reconstruction scan may, e.g., comprise acquiring a CT scan (preferably by a photon counting CT scanner). Acquiring a CT scan may, e.g., comprise acquiring images (e.g., representing image data) of the body of the patient and a subsequent 3-dimensional reconstruction of the body of the patient based on the acquired images, e.g., partially based on the procedure as outlined above.

Subsequently, an operator of the workflow 400 (e.g., a physician and/or any other member of a medical staff) may be supplied 430 with a means (e.g., a button displayed in a user interface used for controlling the workflow 400) for identifying the SMA 110, 210 and/or IMA 120, 220.

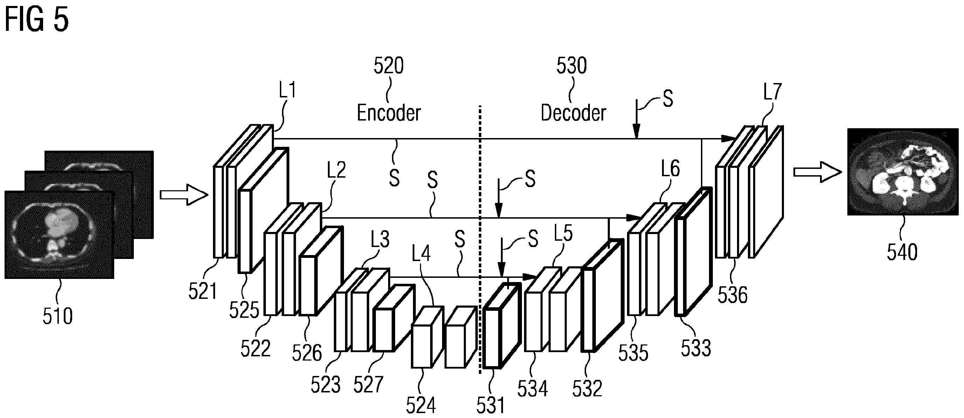

Initiating the identifying of the SMA 110, 210 and/or IMA 120, 220, based on activating the means, may initiate a deep learning workflow comprising the steps 431-435, used to train an artificial intelligence, which will be discussed in further detail below. The artificial intelligence may, in some examples, be provided as described with reference to Fig. 5, below.

In step 431, image data related to, e.g., the abdominal region of the patient may be collected. The collection may comprise acquiring the respective image data, e.g., by means of a CT scanner (e.g., a photon counting CT scanner) and/or retrieving the data from a remote entity such as, e.g., a remote server which may run a remote database.

The image data may relate to a single patient or may relate to different patients. Each image data may depict the SMA 110, 210 and/or IMA 120, 220. In an example, each image data may be associated with acquiring images with a resolution of 1024 x 1024 pixel computed tomography angiography (CTA) coronal multiphase reconstruction/maximum intensity projection images of the abdomen region. Each acquired image may be associated with a layer of thickness 0.2 mm representing a slice of the abdomen of the patient. The slices may be acquired at 0.1 mm increments (e.g., along an axial direction of the body of the patient).

Kernel Bv68 may preferably be used for the artificial intelligence to support the obtaining of sufficiently expressive images of the SMA 110, 210 and/or IMA 120, 220 preferably along with iliac arteries.

In step 420, the image data generated in step 410 may be augmented using data augmentation. In this context, data augmentation may be referred to as an artificial increase of the total amount of image data generated in step 410 by rotating, scaling, flipping and/or cropping the image data obtained in step 410. Consequently, the total amount of potentially available training data may be increased without the necessity for acquiring further CT scans which may support a cost-effective training of the artificial intelligence.

In step 433, the augmented image data may be labeled (e.g., manually and/or based on a pre-trained artificial intelligence). The labeling, when carried out manually, may comprise the manual identification of the SMA 110, 210, IMA 120, 220 and/or terminal branches (111-114 and/or 121-123) thereof by a physician. The labelling may, e.g., be carried out by means of a respective user interface, which may display augmented image data to a physician based on which the physician may select the SMA 110, 210, IMA 120, 220 and/or (one or more of the) terminal branches (111-114 and/or 121-123) thereof. It will be understood that the accuracy of this step of labelling the augmented image data is crucial for the success of the training of the artificial intelligence and to avoid introducing bias into the model to be trained.

Once the augmented image data is labeled to obtain labeled augmented image data, the labeled augmented image data may be split into training data, validation data and test data sets which may subsequently be used to train and evaluate the model underlying the finally trained artificial intelligence.

In an example, the labeled augmented image data may be split into 70% of training data, 15% of validation data and 15% of test data, however, other split ratios may be possible.

In step 434, the split labeled augmented image data may be fed to a U-Net architecture to train the respective U-Net network. A U-Net is an architecture representing a fully convolutional neural network that specializes in image segmentation, also known as semantic segmentation.

Semantic segmentation may be referred to as a procedure which does not only identify the SMA 110, 210 and/or IMA 210, 220 as such (i.e., their sole presence) but may also be able to create a mask superimposing the image data that depicts the SMA 110, 210 and/or IMA 210, 220 such that the mask highlights where in the image data the specific artery is located, preferably along with its dimensions and may thus advantageously support medical image data segmentation. Using the specific case of a U-Net architecture, different masking may be applied for the SMA 110, 210 and/or IMA 210, 220 along with their terminal branches 111-114 and/or 121-123, respectively.

The U-Net architecture may comprise an encoder and a subsequently arranged decoder. This arrangement is described in further detail with reference to Fig. 5, below.

In step 435, after the training of the U-Net architecture has been finished, a validation and test of the trained U-Net architecture may be performed.

For the validation, at least a portion of the validation data set (as described above) may be provided to the at least partially trained U-Net architecture. Based thereon, a further fine-tuning of at least one hyperparameter of the U-Net architecture may be achieved.

The testing of the trained U-Net architecture may be based on providing at least a portion of the training data set to the trained U-Net architecture. Based on providing the test data to the trained U-net architecture, the accuracy of the predictions of the U-Net architecture may be determined.

If said accuracy is considered as sufficient to provide a reliable identification of the SMA 110, 210 and/or the IMA 120, 220, the trained U-Net architecture may be provided with real image data related to a patient. The accuracy may be considered as sufficient if the probability that the trained aritificial intelligence unit erroneously identifies an anatomic structure as the at least one blood vessel (e.g., the SMA 110, 210 and/or the IMA 120, 220) is below a predefined threshold.

That is, in step 440, an identifying of at least one blood vessel of the patient may be performed by providing accessed image data to the trained artificial intelligence unit.

Fig. 5 shows a schematic illustration of the interplay 500 of an encoder 520 and a decoder 530 as it may be used for identifying the at least one blood vessel.

Image data 510 (such as the image data 200), which may, e.g., be provided as input images of matrix size 512 x 512 pixel, may be passed to the encoder 520.

Encoder 520 may comprise one or more layers L1-L4. Each of the layers L1-L4 may comprise two subsequently arranged, e.g., 3 x 3 convolutional layers. The combination of each of the two subsequently arranged convolutional layers may additionally be followed by a batch normalization and an activation function of type ReLu indicated by reference numerals 521-524 in each of the layers L1-L4, respectively.

The batch normalization may improve the speed, performance and stability of the training. Moreover, it may reduce the covariate shift (i.e., the shift of the distribution of input data between the training environment and the live environment).

Subsequent to each of the combination of the two convolutional layers, the batch normalization and the ReLu function (indicated by reference numbers 521-524), preferably except for the very last layer L4 of the encoder 520, a pooling layer 525-527 may be deployed in each of the layers L1-L3 wherein each of the pooling layers is configured to perform a respective pooling operation. Each of the pooling layers 525-527 may contribute to a further dimension reduction of the image data processed by a respective preceding arrangement of the two convolutional layers, the batch normalization and the ReLu function by, e.g., spanning a matrix of, e.g., 2x2 pixel across a representative of the image data, determining the maximum pixel value comprised in said matrix (e.g., a maximum grey scale value, a maximum brightness value, etc.). That is, a 2x2 matrix may be compressed to a single output value only. Since this procedure is applied to the entire representative of the image data, the respective representative may be reduced in its dimension (e.g., its size).

A padding operation of "padding='same'" may be applied to pad evenly left and right to maintain the dimension of the representative of the image data.

At each of the layers L1-L4 in the encoder 520, the size of the image data 510 may be reduced as outlined above. This may be accompanied by an increase of the number of channels/filters (e.g., from 64 to 128 to 256). This may advantageously support a faster learning procedure as each step of the learning procedure may be applied to respectively smaller image sequences of the image data 510.

The implementation of the encoder 520 may be based at least in part on an implementation in Python using the tensorflow package. The implementation may be based at least in part on the methods "Conv2D()", "Activation("relu")" and "MaxPooling2DO".

The encoder 520 may be followed by the decoder 530. Decoder 530 may reverse the dimension reduction of the image data as carried out by the encoder 520. Moreover, decoder 530 may be configured to understand where in the image data 510, initially supplied to the encoder 520 and later on to the decoder 530, the at least one blood vessel are located along with respective dimensions.

In an example, the decoder 530 may be initiated by an upsampling layer 531.

Upsampling layer 531 may be followed by layers L5-L7, wherein at least the layers L5 and L6 (arranged subsequent to the upsampling layer 531) may comprise, e.g., two convolutional layers, preferably 3 x 3 convolutional layers. Each of the two subsequently arranged convolutional layers may additionally comprise a batch normalization and an activation function of type ReLu, arranged subsequent to the convolutional layers, indicated by reference numerals 534 and 535 in each of the layers L5 and L6, respectively.

At each of the layers L5-L7 of the decoder 530, the size of the image data 510 may be increased/reconstructed again, i.e., inverse to the procedure of layers L1-L5 as outlined above. This may be accompanied by a decrease of the number of channels/filters (e.g., from 256 to 128 to 64).

What is more, a stride of 2x2 may be applied and padding="same" may be applied.

The decoder 530 may be implemented in Python and may be based on the keras tensorflow package using at least the functions "Conv2DTranspose()", "concatenateO".

The final layer L7 of the decoder 530 may comprise three convolutional layers 536 (e.g., 3x3 convolutional layers), wherein each of the convolutional layers 536 may be followed by a batch normalization and a ReLu function.

In some cases, the arrangement of the encoder 520 and the decoder 530, as outlined above, may take advantage of skip connections S. By using skip connections S, at least some of the layers L1-L3 of the encoder 520 may be directly connected to respective layers L5-L7 of the same depth of the decoder 530 such that layers from a depth arranged below the depth at which the skipping occurs, are skipped. As outlined above, this may support a more efficient learning procedure as less computation time and memory needs to be allocated for processing the respective layers.

In this context, the upsampling layer 531 may be referred to as lying at a same depth of the encoder 520 and decoder 530 arrangement as layer L4 of the encoder.

The layers L1-L3 of the encoder 520 may be referred to as lying at a same depth of the respective layers L5-L7 of the decoder 530.

As soon as the architecture described above is trained, the underlying model may be compiled using a CrossEntropyLoss function.

The weights associated with the neural network underlying the above-described model are initialized with small random numbers.

Afterwards a processed image data 540 may be output that comprises an indication which indicates the identified at least one blood vessel.

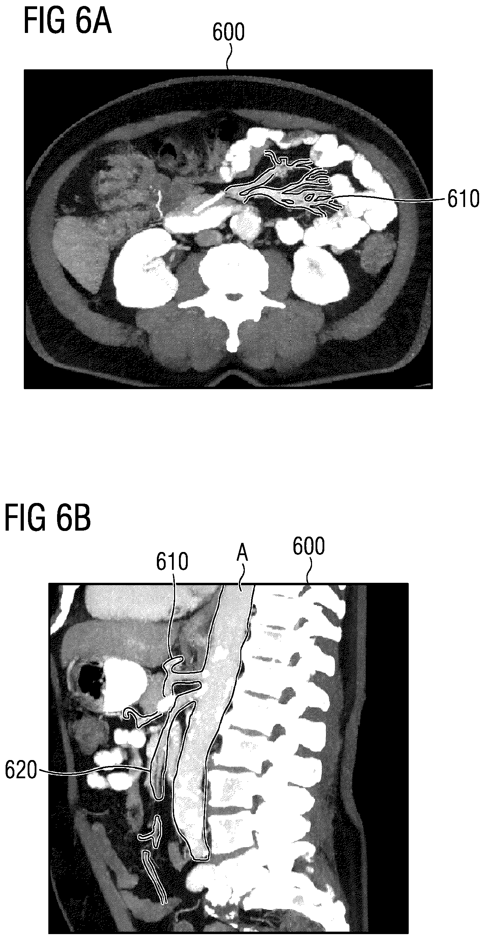

Figs. 6A and 6B show exemplary image data 600 of the SMA 110, 210 and IMA 120, 220 arteries wherein the identified SMA 110, 210 and IMA 120, 220 arteries are visually masked.

Fig. 6A shows abdominal image data 600 of the patient taken in an axial plane of the body of the patient that has been provided with an indication of an identified at least one blood vessel.

An indication has been output that indicates the identified at least one blood vessel (in the present case, the SMA 610 which corresponds to the SMA 110, 210 as discussed with reference to Figs. 1 and 2, above).

In the exemplarily depicted case, the indication has been output as a masking of the original image data (e.g., the original image data 200) by a mask, which in the present case, is provided as a (closed) contour surrounding the identified at least one blood vessel.

Fig. 6B shows abdominal image data 600 taken in a sagittal plane of the body of the patient that has been provided with an indication of an identified at least one blood vessel.

An indication has been output that indicates the identified at least one blood vessel (in the present case, the SMA 610 which corresponds to the SMA 110, 210 and the IMA 620 which corresponds to the IMA 120, 220 as discussed with reference to Figs. 1 and 2, above). Moreover, the indication indicates the abdominal aorta A.

In the exemplarily depicted case, the indication has been output as a masking of the original image data (e.g., the original image data 200) by a mask, which in the present case, is provided as a (closed) contour surrounding the identified at least one blood vessel.

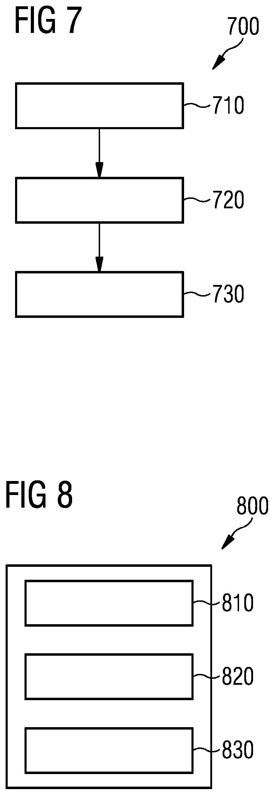

Fig. 7 shows a schematic illustration of a workflow for a method 700 for identifying at least one blood vessel represented in image data.

In step 710, the image data is accessed.

In step 720, the image data is passed to a trained artificial intelligence unit being trained to perform a multi-class segmentation on the image data to identify the at least one blood vessel.

In step 730, an indication is output that is associated with the identified at least one blood vessel.

Fig. 8 shows a schematic illustration of an apparatus 800 for identifying at least one blood vessel represented in image data.

Accessing unit 810 is configured to access the image data.

Passing unit 820 is configured to pass the image data to a trained artificial intelligence unit that has been trained to perform a multi-class segmentation on the image data to identify the at least one blood vessel.

Outputting unit 830 is configured to output an indication associated with the identified at least one blood vessel.

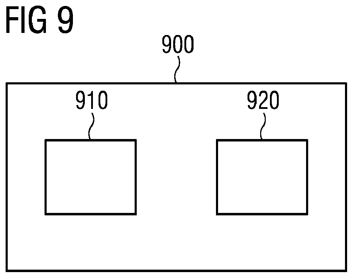

Fig. 9 shows a schematic illustration of a system 900 for identifying at least one blood vessel represented in image data.

Remote storage entity 910 may be configured to store the image data.

The apparatus 920 may be configured as described elsewhere herein.

Although the present invention has been described in accordance with preferred embodiments, it is obvious for the person skilled in the art that modifications are possible in all embodiments.

## Claims

1. A method (700) for identifying at least one blood vessel (110, 120, 210, 220, 111-114, 121-123) represented in image data (200, 510), the method comprising:
accessing (710) the image data (200, 510);
passing (720) the image data (200, 510) to a trained artificial intelligence unit being trained to perform a multi-class segmentation on the image data (200, 510) to identify the at least one blood vessel (110, 120, 210, 220, 111-114, 121-123); and
outputting (730) an indication associated with the identified at least one blood vessel (110, 120, 210, 220, 111-114, 121-123).

2. The method according to claim 1,
wherein accessing the image data (200, 510) comprises retrieving the image data (200, 510) from a remote entity.

3. The method according to claim 1 or 2,
wherein the image data (200, 510) represents abdominal image data.

4. The method according to one of claims 1-3,
wherein the at least one blood vessel (110, 120, 210, 220, 111-114, 121-123) is a superior mesenteric artery, SMA, (110, 210) and/or an inferior mesenteric artery, IMA, (210, 220) and/or a respective terminal branch (111-114, 121-123).

5. The method according to one of claims 1-4,
wherein the image data (200, 510) is a computed tomography, CT, image data.

6. The method according to one of claims 1-5,
wherein the trained artificial intelligence unit is based at least in part on a U-Net architecture.

7. The method according to claim 6,
wherein the U-Net architecture is trained by using skip connections (S).

8. The method according to one of claims 1-7, wherein the outputting (730) comprises:
visually masking the identified blood vessel in the image data (200, 510, 540).

9. The method according to one of claims 1-8, wherein the outputting (730) comprises:
outputting a dimension of the identified at least one blood vessel (110, 120, 210, 220, 111-114, 121-123).

10. The method according to one of claims 1-9, further comprising:
detecting an anomaly in the at least one blood vessel (110, 120, 210, 220, 111-114, 121-123) based at least in part on the outputting (730).

11. A computer program product comprising instructions which, when the program is executed by a computer, cause the computer to carry out the method of one of the claims 1-10.

12. An apparatus (800) for identifying at least one blood vessel (110, 120, 210, 220, 111-114, 121-123) represented in image data, the apparatus comprising:
an accessing unit (810) for accessing the image data (200, 510);
a passing unit (820) for passing the image data to a trained artificial intelligence unit being trained to perform a multi-class segmentation on the image data to identify the at least one blood vessel (110, 120, 210, 220, 111-114, 121-123); and
an outputting unit (830) for outputting an indication associated with the identified at least one blood vessel (110, 120, 210, 220, 111-114, 121-123).

13. The apparatus according to claim 12, further comprising:
an execution unit for executing the method of one of the claims 1-10.

14. The apparatus according to claim 13, further comprising:
an execution unit for executing the program of claim 11.

15. A system (900) for identifying at least one blood vessel (110, 120, 210, 220, 111-114, 121-123) represented in image data (200, 510), the system (900) comprising:
a remote storage entity (910) that is configured to store the image data (200, 510); and
the apparatus (920) according to one of the claims 12-14.
